Europäisches Patentamt

European Patent Office

Office européen des brevets

(19)

(11) Numéro de publication : **0 283 407 B1**

## (12) FASCICULE DE BREVET EUROPEEN

(45) Date de publication du fascicule du brevet :
08.01.92 Bulletin 92/02

(21) Numéro de dépôt : **88420063.5**

(22) Date de dépôt : **23.02.88**

(51) Int. Cl.⁵ : **C08L 83/04, A61K 9/22,**
**C08K 13/02, A61K 33/18,**
**C02F 1/76**

(54) **Composition à base d'élastomère silicone vulcanisable à chaud contenant de l'iode.**

(30) Priorité : 26.02.87 FR 8702882

(43) Date de publication de la demande :
21.09.88 Bulletin 88/38

(45) Mention de la délivrance du brevet :
08.01.92 Bulletin 92/02

(84) Etats contractants désignés :
AT BE CH DE ES FR GB GR IT LI LU NL SE

(56) Documents cités :
DE-A- 1 467 861
GB-A- 1 412 970
US-A- 2 918 400
US-A- 4 275 194
US-A- 4 384 960
US-A- 4 420 590
US-A- 4 640 956

(72) Inventeur : **Cyprien, Guy**
**7, rue d'Anjou**
**F-94240 L'Hay Les Roses (FR)**
Inventeur : **Fisch, Alain**
**22, rue de l'Yvette**
**F-75016 Paris (FR)**
Inventeur : **Haggiage, Johnny**
**72, rue du Fort Saint-Irénée**
**F-69005 Lyon (FR)**
Inventeur : **Porte, Hugues**
**6, Chemin de Crépieux**
**F-69300 Caluire (FR)**
Inventeur : **Prazuck Thierry**
**53, Avenue Mathurin Moreau**
**F-75019 Paris (FR)**
Inventeur : **Torres Ghislaine**
**104, rue Ney**
**F-69006 Lyon (FR)**

(74) Mandataire : **Fabre, Madeleine-France et al**
**RHONE-POULENC CHIMIE Direction des**
**Brevets Secteur Spécialités Chimiques 25,**
**quai Paul-Doumer**
**F-92408 Courbevoie Cédex (FR)**

(73) Titulaire : **RHONE-POULENC CHIMIE**
**25, quai Paul Doumer**
**F-92408 Courbevoie Cédex (FR)**

## Description

La présente invention concerne une composition à base d'élastomère silicone vulcanisable à chaud contenant de l'iode ainsi qu'un procédé de traitement des eaux d'usage domestique et de boissons à l'aide de cette composition. On estime actuellement à plusieurs centaines de millions le nombre de sujets présentant une déficience ou une carence en iode dans le monde. Les zones géographiques les plus touchées sont l'Amérique Latine, en particulier le long de la Cordillière des Andes, la quasi totalité des pays non côtiers de l'Afrique et de l'Asie (Pakistan, Inde, Népal, Chine, Laos, etc.....).

Les principales conséquences pathologiques de la déficience en iode sont bien connues. Il s'agit essentiellement d'une part du goître et ses complications parmi lesquelles on peut citer les troubles de la déglutition, les troubles respiratoires, la cancérisation, la circulation collatérale, et, d'autre part l'hypothyroïdie et ses complications parmi lesquelles on peut citer : le crétinisme, les désordres cérébraux, les accouchements prématurés, les fausses couches et les anomalies congénitales.

Si la carence en iode a disparu des pays industrialisés grâce à l'iodation du sel de cuisine, il n'en est pas de même dans les pays en voie de développement où les deux principales actions menées jusqu'àlors sont demeurées inéfficaces.

Ces actions visent essentiellement d'une part :

— l'iodation du sel de cuisine : elle est inopérante dans la plupart des pays en voie de développement car bien souvent la consommation de sel est minime, les circuits de distribution du sel à travers des réseaux économiques et commerciaux sont quasiment inexistants et, enfin, en région tropicale, l'iode rajouté au sel s'en échappe rapidement lorsqu'il n'est pas parfaitement emballé.

et d'autre part :

— l'injection intra-musculaire d'huile iodée : cette injection a l'avantage de présenter une action différée (action retard) mais elle n'est pas sans présenter des inconvénients, en particulier les risques d'infection, les risques d'allergie à l'iode, les risques d'hyperthyroïdie ou d'hypothyroïdie induites par l'injection d'une dose nécessairement supra-physiologique.

On a par ailleurs décrit dans le brevet belge BE-A-889680 l'introduction d'oligo-éléments, dont l'iode, dans l'eau de boisson des ruminants, sous la forme d'une dispersion dans un liant comme par exemple du plâtre de Paris. Un diorganopolysiloxane peut être ajouté en vue de retarder la diffusion de l'oligo-élément. En outre l'utilisation d'iode et de composés de l'iode pour désinfecter ou pour purifier l'eau est bien connue. On peut citer à titre d'exemple les brevets américains US-A2347567, US-A-2743208 et US-A-3408295.

Il existe également de très nombreux brevets décrivant l'utilisation de systèmes polymères, en particulier de silicone, pour la libération contrôlée d'un principe actif au moyen par exemple d'un système transdermique (brevet américain US-A-4053580), ou par absorption buccale notamment pour des ruminants (brevet français FR-A-2560768).

Enfin par le brevet américain US-A-4384960 il est décrit la mise en pastilles d'iode $I_2$ dans une bouteille en plastique dans laquelle l'eau pénètre par une membrane poreuse de polymère. L'eau solubilise l'iode. Le rôle de la membrane est seulement d'éviter que l'iode en pastilles ne sorte de la bouteille.

Il est simplement suggéré en outre qu'il est possible d'introduire l'iode $I_2$ dans la bouteille au sein d'une dispersion liquide de silicone ou d'un élastomère de diméthylsiloxane puis de les faire durcir. Cette solution indiquée n'est pas techniquement réalisable car d'une part $I_2$ est un inhibiteur bien connu des catalyseurs de durcissement des élastomères silicones vulcanisables à température ambiante (voir en particulier la publication de W.D. MORAIN et al. Plastic and Reconstructive Surgery 59, 2, 215-222 (1977)), et d'autre part du fait de sa volatilité élevée, $I_2$ se sublime lors de la réticulation à chaud des élastomères silicones.

Toutefois dans ce système il n'y a pas de contrôle de la libération de l'iode d'une part et, d'autre part, l'iodation de l'eau se fait par addition discontinue ou en continu de quelques gouttes d'eau très iodée (à saturation) contenue dans la bouteille, dans un récipient quelconque contenant de l'eau non traitée. Il est clair que la solution proposée par US-A-4384960 est imparfaite, par le fait notamment qu'il s'agit d'une technique individuelle qui, comme l'injection intra-musculaire d'iode, nécessite une éducation et une mobilisation massive des populations.

Le brevet US-A-4420590 enseigne que de l'eau peut être rendue potable après passage au contact d'une résine échéangeuse d'anion traitée par essentiellement :

— de l'iode élémentaire,
— du bromure de potassium,
— de l'iodure de potassium,
dans des proportions relatives bien définies.

Le brevet US-A-4384960 enseigne qu'il est possible de prévenir les conséquences d'une carence en iode en ajoutant de l'iode élémentaire (c'est-à-dire au degré d'oxydation 0) à de l'eau.

Le brevet anglais GB-A-1412970 enseigne que des substances pharmacologiquement actives, non ioniques et lipophiles, peuvent être libérées dans l'organisme après qu'elles aient été mélangées avec un élastomère silicone vulcanisant à froid, le produit résultant de ce mélange étant mis sous la forme filamentaire et introduit dans l'organisme ou mis à son contact.

La présente invention a précisément pour but de proposer une composition de silicone contenant de l'iode utilisable pour le traitement continu des eaux d'usage domestique, en particulier dans les systèmes d'adduction et de traitement des eaux dans les puits et les forages et qui permette de relarguer (libérer) une quantité contrôlée et adaptée d'iode en vue d'assurer d'une part le traitement collectif des diverses manifestations dues à une carence en iode et d'autre part une prophylaxie de ces diverses manifestations.

Elle a également pour but de proposer une composition de silicone contenant de l'iode qui immergée de façon appropriée dans les endroits contenant l'eau à traiter, en particulier les puits et forages, relargue (libère) de façon continue, de préférence pendant au moins un an, une quantité appropriée d'iode sous une forme et à une dose thérapeutiquement active et efficace pour soigner les diverses maladies dues à une carence en iode.

Elle a également pour but de proposer une composition élastomère de silicone contenant de l'iode qui, immergée de façon appropriée dans les endroits contenant l'eau à traiter n'ait aucune action secondaire indésirable et néfaste sur le plan chimique et biologique sur les eaux à traiter.

Elle a également pour but de proposer une composition élastomère de silicone contenant de l'iode présentant une forme adaptée au milieu dans lequel se trouve l'eau à traiter, cette forme étant adaptée en particulier aux puits et/ou forages et présentant un système d'introduction dans les puits et/ou forages permettant son remplacement aisé.

Ces buts et d'autres sont atteints par la présente invention qui concerne en effet une composition de silicone vulcanisable à chaud comportant :

(A) une gomme diorganopolysiloxane présentant, par molécule au moins deux groupes vinyle liés au silicium et une viscosité à 25°C d'au moins 500000 mPa · s,

(B) au moins un organohydrogénopolysiloxane présentant par molécule au moins trois atomes d'hydrogène liés au silicium,

(C) une charge renforçante,

(D) une quantité catalytiquement efficace d'un catalyseur qui est un composé d'un métal du groupe du platine et

(E) au moins un composé de l'iode à l'exclusion de l'iode moléculaire $I_2$, solide à la température ambiante, soluble dans l'eau, non toxique, dispersé de façon homogène dans la composition.

Comme composé minéral de l'iode, à l'exception de l'iode moléculaire $I_2$, on peut utiliser seul ou en mélange :

— les iodures ou iodates de formules générales

$$(M^{a+}) (I^-)a$$
$$\text{et} \quad (M^{a+}) (IO_3^-)a$$

dans lesquelles a est un nombre entier supérieur ou égal à 1 et M est un cation qui peut être choisi parmi un métal alcalin tel que le sodium et le potassium, un métal alcalino-terreux tel que le magnésium et le calcium, un métal de transition tel que le fer et le manganèse, un ammonium quaternaire $(NY_4)^+$, dont les radicaux Y identiques ou différents représentent un radical alkyle linéaire ou ramifié en $C_1$-$C_{20}$ ou un atome d'hydrogène, tel que l'ion ammonium $NH^+_4$

Les cations $M^{a+}$ et $NY_4^+$, sont choisis de telle sorte que l'iodure ou l'iodate correspondant soit un solide ou un liquide à température ambiante, soit soluble dans l'eau et soit non toxique.

Comme iodures et iodates on peut en particulier utiliser ceux de formules :

NaI,      NaIO$_3$,
KI,      KIO$_3$,
MgI$_2$,      MgI$_2$ · 8H$_2$O,
Mg(IO$_3$)$_2$ · 4H$_2$O,
NH$_4$I
FeI$_2$ · 4H$_2$O
MnI$_2$

Ces sels peuvent contenir de l'eau d'hydratation ou de l'eau de constitution.

Pour des raisons de facilité de mise en oeuvre les composés d'iode solides sont préférés, et parmi ceux-ci

NaI et KIO$_3$ sont les plus préférés.

Tous les composés de l'iode (E) tels que définis ci-dessus, lorsqu'ils sont en solution dans l'eau à traiter, libèrent l'iode sous une forme non toxique et thérapeutiquement efficace. Par composé de l'iode non toxique on entend selon l'invention un composé qui, en solution, n'est pas toxique aux posologies préconisées par la présente invention.

Par composé de l'iode soluble dans l'eau on entend un composé présentant une solubilité d'au moins 100 µg/l à température ambiante.

On utilise généralement de 5 à 150 parties de préférence de 10 à 100 parties de composé de l'iode (E) pour 100 parties de gomme (A).

En particulier dans les pays en développement, l'eau à usage domestique (boisson, lavage, irrigation, etc.....) est pour l'essentiel apportée par deux types de structure, les puits et les forages.

Pour des raisons évidentes de coût, d'efficacité, de salubrité, la création nouvelle d'un point d'eau se fait souvent par forage.

Un forage est une colonne d'air forée à travers des roches compactes ayant une profondeur comprise généralement entre 20 et 100 mètres et un diamètre d'au moins environ 10 cm. L'eau s'infiltre dans cette colonne, par les fissures ou les divers interstices. La réserve d'eau immédiatement disponible est donc constituée d'une colonne de 10 à 70 mètres, généralement de 30 à 50 mètres de haut qui est extraite à l'aide d'une pompe à corps immergé.

Cette eau se renouvelle principalement en fonction de l'utilisation du forage qui dépend de la saison. En effet en saison des pluies le forage est traditionnellement moins utilisé. Par contre en saison sèche, le forage débite environ 10-12 heures par jour, soit une quantité comprise entre 5 et 10 m$^3$ par jour pendant environ six mois.

En règle générale, un puits peut être asséché deux fois durant la journée au moment de la saison sèche ce qui correspond avec ces données statistiques moyennes, à un maximum d'utilisation de 5 à 10 m$^3$.

De nombreuses études montrent que dans les zones de grande endémie goîtreuse, le taux pré-existant d'équivalent en iode dans l'eau des forages ou des puits est inférieur à 2 microgrammes par litre (2 µg/l). Il est estimé actuellement qu'un apport journalier d'envrion 100 µg d'équivalent iode par jour et par personne serait suffisant pour prévenir le développement d'un goître endémique et sans doute environ 150 µg lorsqu'il y a consommation régulière de substances goitrigènes. A l'inverse une intoxication aigüe à l'iode peut être responsable d'irritation neurologique, d'hyperthyroïdie ou d'hypothyroïdie.

Il est admis en pratique médicale que l'absorption d'une dose de 3 grammes d'équivalent d'iode par un sujet adulte en une seule prise n'entraîne pas d'effet secondaire.

Par conséquent, pouvoir apporter à un individu de 20 à 200 µg de préférence environ 100 µg, d'équivalent iode par jour constitue le but recherché.

Ainsi sachant qu'un individu adulte absorbe en moyenne 2 litres d'eau par jour et sur la base des données ci-dessus (forage ayant un débit de 600 l/h), il apparaît souhaitable qu'un litre d'eau traitée contienne environ 50 µg/l d'iode ce qui correspond à 50 µg d'équivalent iode par litre et par personne, ce qui nécessite que la composition de silicone relargue 720 mg/j d'équivalent d'iode, soit 270 g d'équivalent iode à relarguer pendant une année.

Dans ce qui suit ou ce qui précède, sauf indications contraires, les parties et pourcentages sont en poids.

De façon surprenante et inattendue on a en effet découvert selon la présente invention qu'il est possible d'ajouter dans une composition élastomère silicone vulcanisable à chaud des quantités importantes de composé d'iode sous une forme solide ou liquide tel que défini ci-dessus, à savoir p.ex. de 5 à 130 parties, de préférence de 20 à 100 pour 100 parties de la somme des organopolysiloxane (A) + (B). Cette composition est préalablement chargée avec une charge renforçante et on obtient ainsi, après réticulation à chaud un élastomère qui présente des caractéristiques mécaniques suffisantes pour l'application envisagée et qui, immergé dans l'eau permette d'assurer un relargage continu et contrôlé d'iode de préférence pendant au moins un an.

Le système de libération contrôlée de l'iode fait partie des systèmes matriciels dont la diffusion du principe actif est normalement régie par la loi de FICK, c'est-à-dire par une cinétique de diffusion d'ordre 1/2 pour seulement 60% en poids du principe actif. Au-delà de 60% il y a épuisement de la matrice et les flux de diffusion sont fortement diminués. De façon surprenante et inattendue, on a trouvé que le système matriciel silicone conforme à l'invention libère l'iode suivant une cinétique d'ordre 0 et de façon continue et cela jusqu'à ce que 80% en poids et plus du composé d'iode soit libéré.

L'avantage considérable apporté par la matrice silicone est donc qu'il est très facile d'extrapoler la diffusion continue du principe actif après une mesure de la quantité libérée au bout d'au moins un mois puisque l'on sait que la cinétique de diffusion est d'ordre 0 et qu'au moins 80% du composé d'iode sera libéré suivant cette cinétique.

De façon à maîtriser la libération du principe actif, il est avantageux de présenter la matrice silicone sous la forme de modules (éléments) élémentaires de formes variées tels que des cubes, des parallélépipèdes rectangles, des cylindres, des sphères dont les paramètres fondamentaux sont les suivants :

— la nature du composé d'iode,

— le diamètre moyen (granulométrie) g des particules du composé d'iode dans dans le cas préféré où ce dernier est un solide,

— la teneur de composé d'iode au sein de la matrice,

— le rapport R de la surface au volume du module.

La nature du composé d'iode et sa granulométrie définissent la vitesse de diffusion à travers la matrice, du principe actif.

Plus g est petit et plus v est lent et inversement.

Plus t est grand et plus le flux de principe actif est élevé et inversement.

Plus R est grand et plus le flux élevé de principe actif est grand et inversement.

L'homme de métier, par des expériences de routine, peut sans difficulté aboutir rapidement au résultat visé en extrapolant le temps d'élution théorique qui correspondra au temps réel de diffusion du principe actif.

Pour NaI et KIO$_3$ qui sont les composés d'iode préférés, g, t et R peuvent être avantageusement choisis dans les zones suivantes :

— g compris entre 1 et 300 μm,

— t compris entre 10 et 100 parties en poids de composé d'iode pour 100 parties de (A),

— R compris entre 0,5 et 50 pour une forme cylindrique.

Le composé d'iode est dispersé de façon homogène au sein de la matrice.

Plus précisément la présente invention concerne une composition de silicone comportant :

— (A) : 100 parties d'une gomme diorganopolysiloxane ayant une viscosité présentant par molécule au moins deux groupes vinyle liés au silicium et une viscosité à 25°C d'au moins 500000 mPa · s,

— (B) : au moins un organohydrogénopolysiloxane présentant par molécule au moins 3 atomes d'hydrogène, liés au silicium, en quantité telle que le rapport en nombre des fonctions hydrure de (B) sur les groupes vinyle de (A) soit compris entre 0,4 et 10,

— (C) : 5 à 130 parties d'une charge renforçante de préférence siliceuse choisie parmi les silices de pyrogénation et les silices de précipitation,

— (D) : une quantité catalytiquement efficace d'un catalyseur qui est un composé d'un métal du groupe du platine, et

— (E) : 5 à 150 parties d'au moins un composé de l'iode à l'exclusion de l'iode moléculaire I$_2$, solide à la température ambiante, soluble dans l'eau, non toxique, dispersé de façon homogène dans la composition.

Le rapport en nombre des fonctions hydrures de (B) sur les fonctions vinyle de (A) peut être très variable. Il est généralement compris entre 0,4 et 10, de préférence entre 1,1 et 4.

Plus particulièrement la gomme diorganopolysiloxane (A) a pour formule générale R$_{3-a}$(R'O)$_a$SiO(R$_2$SiO)$_n$Si(OR')$_a$R$_{3-a}$ dans laquelle les symboles R, identiques ou différents, représentent des radicaux hydrocarbonés en C$_1$-C$_8$, substitués ou non par des atomes d'halogène, des radicaux cyano ; le symbole R' représente un atome d'hydrogène, un radical alkyle en C$_1$-C$_4$, le symbole a représente zéro ou un, le symbole n représente un nombre ayant une valeur suffisante pour obtenir une viscosité d'au moins 500000 mPa · s à 25°C, au moins 50% en nombre de radicaux représentés par R sont des radicaux méthyle.

De préférence 0,005 à 0,5 mole % des motifs entrant dans la constitution de la gomme (A) sont choisis parmi ceux de formules (CH$_2$ = CH)(R)SiO et/ou (CH$_2$ = CH)R$_{2-a}$(RO')$_a$SiO$_{0,5}$.

La gomme (A) de viscosité de 500000 mPa · s à 25°C, de préférence d'au moins 1 Million de mPa · s à 25°C, est constituée, le long de sa chaîne, de motifs R$_2$SiO et elle est bloquée à chaque extrémité de sa chaîne par un motif R$_{3-a}$R(O)$_a$SiO$_{0,5}$ ; cependant la présence en mélange avec ces motifs, de motifs de structure différente, par exemple de formule RSiO$_{1,5}$ et SiO$_2$, n'est pas exclue dans la proportion d'au plus 2% par rapport au nombre total des motifs R$_2$SiO et R$_{3-a}$(RO')$_a$SiO$_{0,5}$.

Le symbole R représente un radical hydrocarboné en C$_1$-C$_8$ substitué ou non par des atomes d'halogène, des radicaux cyano ; il englobe plus spécifiquement :

— des radicaux alkyle en C$_1$-C$_5$, substitués ou non par des atomes d'halogène, des radicaux cyano, tels que les radicaux méthyle, éthyle, propyle, isopropyle, butyle, isobutyle, pentyle, trifluoro-3,3,3 propyle, β-cyanoéthyle, γ-cyanopropyle,

— des radicaux alcényle en C$_2$-C$_4$ tels que les radicaux vinyle, allyle, butène-2 yle,

— des radicaux aryle mononucléaire en C$_6$-C$_8$, substitués ou non par des atomes d'halogène tels que les radicaux phényle, chlorophényle, tolyle, trifluorométhylphényle.

Les radicaux alkyle en C$_1$-C$_4$, représentés par le symbole R', concernent plus spécifiquement les radicaux méthyle, éthyle, propyle, isopropyle, butyle, butyle secondaire.

Au moins 50% en nombre, de préférence au moins 70% des radicaux représentés par R sont des radicaux méthyle.

Par ailleurs des radicaux vinyle sont également présents, en quantité appropriée, dans la gomme (A) ; ils conduisent à des motifs de formule $CH_2 = CH(R)SiO$ et $CH_2 = CH(R_{2-a})(R'O)_aSiO_{0,5}$ dont le nombre représente 0,005 à 0,5 mole %, de préférence 0,01 à 0,45 mole %, de l'ensemble des motifs de formules générales $R_2SiO$ et $R_{3-a}(R'O)_aSiO_{0,5}$, entrant dans la constitution de la gomme (A).

A titre d'exemples concrets de motifs constituant les gommes (A) peuvent peuvent être cités ceux de formules :

$(CH_3)_2SiO$,
$CH_3(CH_2 = CH)SiO$,
$CH_3(C_6H_5)SiO$,
$(C_6H_5)_2SiO$,
$CH_3(C_2H_5)SiO$,
$CH_3CH_2-CH_2(CH_3)SiO$,
$CH_3(n \cdot C_3H_7)SiO$,
$(CH_3)_3SiO_{0,5}$,
$(CH_3)_2CH_2 = CHSiO_{0,5}$,
$CH_3(C_6H_5)_2SiO_{0,5}$,
$CH_3(C_6H_5)(CH_2 = CH)SiO_{0,5}$,
$HO(CH_3)_2SiO_{0,5}$,
$CH_3O(CH_3)_2SiO_{0,5}$,
$C_2H_5O(CH_3)_2SiO_{0,5}$,
$n \cdot C_3H_7O(CH_3)_2SiO_{0,5}$,
$HO \cdot (CH_2 = CH)(CH_3)SiO_{0,5}$.

Les gommes (A) sont commercialisées par les fabricants de silicones, d'autre part elles peuvent être aisément fabriquées en mettant en oeuvre des techniques abondamment décrites dans la littérature chimique.

Dans la majorité des cas, on utilise des gommes méthylvinyldiméthylpolysiloxanes possèdant le long de leur chaîne des motifs $(CH_3)_2SiO$ et $CH_2 = CH(CH_3)SiO$ et à l'extrémité de leur chaîne des motifs choisis parmi ceux de formules :

$(CH_3)_2CH_2 = CHSiO_{0,5}$, $HO(CH_3)(CH_2 = CH)SiO_{0,5}$,
$(CH_3)_3SiO_{0,5}$, $C_6H_5(CH_3)CH_2 = CHSiO_{0,5}$,
$HO(CH_3)_2SiO_{0,5}$,

ou des gommes diméthylpolysiloxanes bloquées à chaque extrémité de leur chaîne par l'un des motifs précédents renfermant un radical vinyle.

Elles présentent généralement une viscosité d'au moins 2 Millions de mPa · s à 25°C.

L'organohydrogénopolysiloxane (B) présente un motif siloxane de formule générale moyenne :

$$(H)_c \, (R'')_d \, SiO_{\frac{4-d-c}{2}}$$

dans laquelle R″ représente des radicaux méthyle, phényle et vinyle, 50% au moins de ces radicaux étant des radicaux méthyle, c représente un nombre quelconque de 0,01 à 1 inclus et d représente un nombre quelconque de 0,01 à 2 inclus.

Ces organohydrogénopolysiloxanes (B) sont choisis parmi les polymères linéaires, ramifiés ou cycliques, constitués de motifs choisis parmi ceux de formules :

$R''_2SiO$, $H(R'')SiO$, $H(R'')_2SiO_{0,5}$, $HSiO_{1,5}$, $R''SiO_{1,5}$, $SiO_2$, $R''_3SiO_{0,5}$.

Ils peuvent être liquides, gommeux ou résineux. Ils renferment au moins 3SiH par molécule.

Des exemples concrets de produits (B) abondamment cités dans la littérature sont décrits en détail dans les brevets américains US-A-3220972, US-A-3284406, US-A-3436366 et US-A-3697473 cités comme références.

Les charges (C) qui sont de préférence des silices renforçantes sont utilisées à raison de 5 à 130 parties

6

des gommes diorganopolysiloxanes (A). Elles sont choisies parmi les silices de combustion et les silices de précipitation. Elles ont une surface spécifique, mesurée selon les méthodes BET, d'au moins 50 m²/g, de préférence supérieure à 70 m²/g, une dimension moyenne des particules primaires inférieure à 0,1 µm (micromètre) et une densité apparente inférieure à 200 g/litre.

Ces silices peuvent être incorporées telles quelles ou après avoir été traitées par des composés organosiliciques habituellement utilisés pour cet usage. Parmi ces composés, figurent les méthylpolysiloxanes tels que l'hexaméthyldisiloxane, l'octaméthylcyclotétrasiloxane, des méthylpolysilazanes tels que l'hexaméthyldisilazane, l'hexaméthylcyclotrisilazane, des chlorosilanes tels que le diméthyldichlorosilane, le triméthylchlorosilane, le méthylvinyldichlorosilane, le diméthylvinylchlorosilane, des alcoxysilanes tels que le diméthyldiméthoxysilane, le diméthylvinyléthoxysilane, le triméthylméthoxysilane. Lors de ce traitement, les silices peuvent accroître leur poids de départ jusqu'à un taux de 20%, de préférence 18% environ.

Il est souhaitable d'ajouter une quantité catalytiquement efficace d'un catalyseur (D) d'hydrosilylation qui est un composé du groupe du platine, de préférence le platine à la dose de 0,001 à 1%, de préférence de 0,05 à 0,5% calculés en poids de métal catalytique par rapport au poids de la gomme (A) et de l'organohydrogénopolysiloxane (B).

Tous les composés du platine abondamment décrits dans la littérature comme catalyseurs d'hydrosilylation sont utilisables, en particulier l'acide chloroplatinique $H_2PtCl_6$, les produits de réaction de l'acide chloroplatinique avec des alcools, des éthers ou des aldéhydes (brevet américain US-A-3220972) et les produits de réaction de l'acide chloroplatinique avec des vinylpolysiloxanes, traités ou non par un agent alcalin pour éliminer au moins partiellement les atomes de chore (brevets américains US-A-3419593, US-A-3775452 et US-A-3814730).

On peut remplacer jusqu'à 90% en poids des silices de renforcement (C) par des charges semi-renforçantes ou de bourrage dont le diamètre particulaire est supérieur à 0,1 µm, telles que le quartz broyé, les argiles calcinées et les terres de diatomées.

Les compositions de silicone peuvent comporter en outre de 1 à 10 parties d'huiles diméthylpolysiloxanes (F) à extrémités silanol de viscosité à 25°C comprise entre 10 et 5000 mPa · s, de préférence de 30 à 1000 mPa · s, pour 100 parties de gomme (A). Leur utilisation est surtout recommandée lorsque les quantités de charges renforçantes (C) sont élevées.

La préparation des compositions conformes à l'invention s'effectue à l'aide de moyens mécaniques connus, par exemple de pétrins, de mélangeurs à cylindres, de mélangeurs à vis.

Les divers constituants sont incorporés dans ces appareils dans un ordre pouvant être quelconque. Il est toutefois recommandé de charger la gomme (A) puis dans l'ordre les charges siliceuses (C) et le composé d'iode (E), éventuellement l'additif (F) et, en dernier lieu, le composé (D). Si la composition doit être stockée avant son extrusion et/ou moulage, il est souhaitable d'ajouter une quantité efficace d'un inhibiteur de l'action catalytique du platine qui disparaît à chaud lors de la vulcanisation de la composition. On peut ainsi utiliser comme inhibiteur, en particulier les amines organiques, les silazanes, les oximes organiques, les diesters de diacides carboxyliques, les cétones acétyléniques, les vinylméthylcyclopolysiloxanes (voir notamment US-A-3445420 et US-A-3989667). L'inhibiteur est utilisé à raison de 0,005 à 5 parties, de préférence 0,01 à 3 parties pour 100 parties de constituant (A).

Les compositions obtenues se moulent et s'extrudent aisément ce qui permet de réaliser des formes très variées. Elles sont en outre durcissables en élastomères par chauffage sous pression ou à l'air ambiant à des températures de l'ordre de 100 à 350°C. La durée du chauffage varie évidemment avec la température, la pression et la nature des réticulants. Elle est généralement de l'ordre de plusieurs minutes vers 150-250°C et de quelques secondes vers 250-350°C.

Les élastomères ainsi formés peuvent être éventuellement post-chauffés ultérieurement, surtout ceux obtenus par moulage pendant une période d'au moins une heure à une température comprise entre 190 et 270°C dans le but d'achever leur réticulation.

Néanmoins, ces élastomères possèdent dès la fin de leur première phase de réticulation, c'est-à-dire avant la phase éventuelle de post-chauffage, des caractéristiques physiques suffisantes pour l'application envisagée.

Les compostions de silicones selon l'invention sont tenues immergées dans les eaux à traiter suivant une quantité telle que ces compositions assurent un relargage (une libération) continu et contrôlé, de préférence pendant au moins un an.

Ainsi les compositions selon l'invention peuvent être malaxées à froid telles quelles et être extrudées et/ou moulées puis vulcanisées sous forme de modules (éléments) unitaires ; on peut par exemple extruder la composition sous la forme d'un cylindre de diamètre compris entre 0,5 et 9 cm. Les cylindres de composition de silicone obtenues peuvent être découpées dans le cas de leur utilisation dans les forages à la longeur désirée de manière à ce que le cylindre ou les modules découpés à partir du cylindre comportent une quantité suffisante en équivalent iode pour un relargage de préférence pendant au moins une année. Au bout de cette

7

période les cylindres sont remplacés.

De façon surprenante on a découvert que ces compositions de silicone vulcanisables à chaud ont des caractéristiques physiques suffisantes pour les applications envisagées et relarguent l'iode de façon continue et contrôlée.

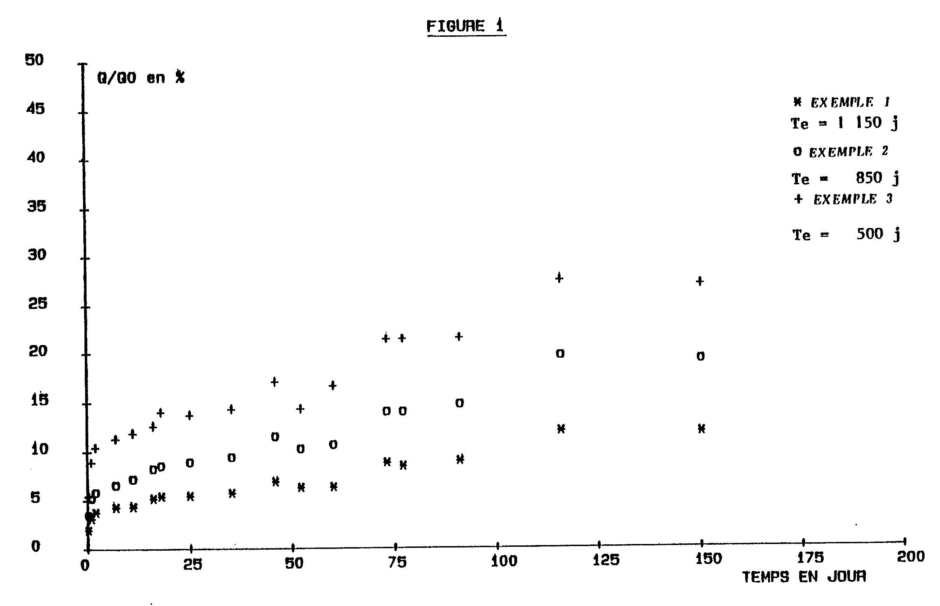

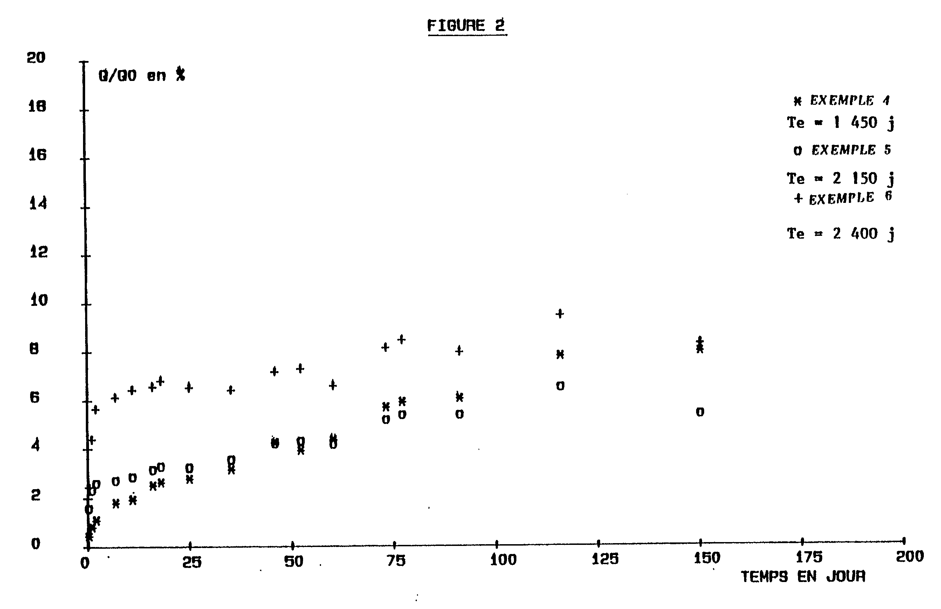

Les exemples ci-après illustrent l'invention sans en limiter sa portée. Référence sera faite au dessin annexé sur lequel les figures 1 et 2 donnent la quantité libérée d'iode par rapport à la quantité initiale en fonction du temps.

Exemple 1.

Préparation de la composition.

Composition : on mélange intimement à l'aide d'un malaxeur les ingrédients suivants :
* 100 parties d'une gomme (A) diméthylméthylvinylpolysiloxane bloquée à chacune de ses deux extrémités par un motif triméthylsiloxy et comprenant dans sa chaine 99,8% molaire de motif diméthylsiloxy et 0,2% molaire de motifs vinylméthylsiloxy et de viscosité 10 millions de mPa · s à 25°C.
* 43,5 parties de charge (B) qui est une silice de combustion traité D4 (octaméthylcyclotetrasiloxane) de surface spécifique BET de 300 m2/g.
* 1 partie d'un diméthylpolysiloxane linéaire bloqué à ses deux extrémités par des groupements diméthylhydroxysiloxane de viscosité 50 mPa · s.
* 0,2 partie d'octométhyltétracyclosiloxane).
* 37,2 parties de NaI de granulométrie moyenne égale à 5 µm (C).

Le malaxage est arrêté 30 minutes après la fin de l'introduction de la silice. On enlève du malaxeur la composition homogène venant d'être préparée et qui est appelée le mélange maître (MM).

On transfère le MM sur un mélangeur à cylindres pour incorporer pour 100 parties de MM :
* 0,43 partie d'un méthylhydrogénosiloxane linéaire de viscosité 45 mPa · s à 25°C, bloqué à chaque extrémité de sa chaine par un motif triméthylsiloxy, comportant dans sa chaine essentiellement des motifs hydrogénosiloxy.
* 0,6 partie d'un empâtage d'acide hexachloroplatinique titrant 0,18% en poids de platine métal.

La composition catalysée se détache aisément des cylindres du mélangeur. Elle est ensuite extrudée en un boudin continu de 26 mm de diamètre qui est ensuite durci par passage dans un four de 6 m porté à 350°C à la vitesse de 1 m par minute. On obtient un jonc uniforme qui est ensuite recuit à 150°C pendant 4 heures.

Protocole expérimental de la mesure de la cinétique d'élution.

La composition élastomère contenant le NaI est découpé à la longueur désirée (30,5 mm), conformément au rapport Surface/Volume (2,14 cm$^{-1}$) que l'on désire atteindre et immergée dans un récipient de 500 ml d'eau distillée, thermostatée à 20°C.

Le récipient est équipé d'un système d'agitation magnétique, animé d'un mouvement de rotation lent (100 tr/min) assurant l'homogénéité de la solution. Il est recouvert d'un couvercle afin de minimiser l'évaporation de l'eau.

Des prélévements journaliers de 1 ml sont réalisés dans les premiers temps de l'élution, et hebdomadaires au bout de 15 jours d'élution.

La concentration en iodure ou iodate, libérée par jour, est déterminée par un dosage avec une électrode spécifique à iodure :

A un millilitre de prélévement du récipient on rajoute deux millilitres d'une solution (K2SO4 + acide ascorbique) — cette solution joue le rôle de tampon ionique, et de solution réductrice dans le cas du dosage des iodates — et un millilitre d'eau distillée. On immerge l'électrode dans cette solution et on lit le potentiel électrochimique de la solution. Un courbe d'étalonnage préalablement établie avec des solutions en iodure de 5.10-5 M/l (M Mole) à 5.10-2 M/l permet de calculer la concentration (C) en iodure ou iodate en mg/l de la solution.

Les caractéristiques du cylindre immergé sont :

| | |
|---|---|
| * Diamètre : | 26,4 mm |
| * Hauteur : | 30,5 mm |
| * Surface : | 36,2 cm2 |
| * Volume : | 16,90 cm3 |
| * S/V : | 2,14 cm$^{-1}$ |
| *Masse totale : | 16,67 g |

8

\*Quantité initiale de I(Qo) :       2,82 g

Dans le tableau 1 sont rassemblés les résultats de la cinétique d'élution.

Q correspond à la quantité d'équivalent I (que nous appelons ion actif) éluée à l'instant t.

Sachant que 80% de l'ion actif incorporé élue selon une cinétique d'ordre zéro par rapport au temps, nous pouvons calculer pour chaque exemple le Temps d'élution théorique (Te) selon :

$$Te = \frac{0,8 \times Qo}{Flux\ journalier} \quad (jours)$$

La courbe Q/Qo = f(t), ainsi que le Te, sont reportés sur la figure 1.

## Exemple 2

On utilise la même composition qu'à l'exemple 1 sauf que l'on incorpore 37,2 parties de NaI de granulométrie comprise entre 100 et 200 μm.

Le malaxage est arrêté 30 minutes après la fin de l'introduction de la silice. On enlève du malaxeur la composition homogène venant d'être préparée et qui est appelée le mélange maître (MM).

On transfère le MM sur un mélangeur à cylindres pour incorporer pour 100 parties de MM :

* 0,43 partie d'un méthylhydrogénosiloxane linéaire de viscosité 45 mPa · s à 25°C, bloqué à chaque extrémité de sa chaine par un motif triméthylsiloxy, comportant dans sa chaine essentiellement des motifs hydrogénosiloxy.

* 0,6 partie d'un empâtage d'acide hexachloroplatinique titrant 0,18% en poids de platine métal.

TABLEAU 1

| TEMPS (JOUR) | Q cumulé (gramme) ion acti | 100*Q/Q0 % |
|---|---|---|
| 0.19 | 0.055 | 1.95 |
| 0.98 | 0.088 | 3.11 |
| 2.01 | 0.106 | 3.76 |
| 6.92 | 0.120 | 4.25 |
| 11.03 | 0.121 | 4.29 |
| 15.92 | 0.145 | 5.13 |
| 17.92 | 0.151 | 5.35 |
| 24.97 | 0.152 | 5.40 |
| 35.20 | 0.160 | 5.68 |
| 45.92 | 0.193 | 6.84 |
| 52.23 | 0.176 | 6.23 |
| 60.19 | 0.177 | 6.26 |
| 73.21 | 0.248 | 8.79 |
| 77.14 | 0.238 | 8.43 |
| 91.13 | 0.253 | 8.95 |
| 115.92 | 0.338 | 11.97 |
| 150.18 | 0.334 | 11.84 |

La composition catalysée se détache aisément des cylindres du mélangeur. Elle est ensuite extrudée en un boudin continu de 26 mm de diamètre qui est ensuite durci par passage dans un four de 6 m porté à 350°C à la vitesse de 1 m par minute. On obtient un jonc uniforme qui est ensuite recuit à 150°C pendant 4 heures.

On découpe ensuite un cylindre dont les caractéristiques sont les suivantes :

* Diamètre :                25,9 mm
* Hauteur :                 34,0 mm
* Surface :                 38,2 cm$^2$
* Volume :                  17,90 cm$^3$
* S/V :                     2,14 cm$^{-1}$
* Masse totale :            20,95 g
* Quantité initiale de I(Qo) :  3,45 g

Le dit cylindre est immergé dans 500 ml d'eau distillée,thermostatée à 20°C.

Le tableau 2 rassemble les résultats de la cinétique d'élution.

La courbe Q/Qo = f(t), ainsi que le Te, sont reportés sur la figure 1.

## Exemple 3

On utilise la même composition qu'à l'exemple 1 sauf que l'on incorpore 37,2 parties de NaI de granulométrie comprise entre 200 et 400 µm.

Le malaxage est arrêté 30 minutes après la fin de l'introduction de la silice. On enlève du malaxeur la composition homogène venant d'être préparée et qui est appelée le mélange maître (MM).

On transfère le MM sur un mélangeur à cylindres pour incorporer pour 100 parties de MM :

* 0,43 partie d'un méthylhydrogénosiloxane linéaire de viscosité 45 mPa · s à 25°C, bloqué à chaque extrémité de sa chaine par un motif triméthylsiloxy, comportant dans sa chaine essentiellement des motifs hydrogénosiloxy.

## TABLEAU 2

| TEMPS (JOUR) | Q cumulé (gramme) ion acti | 100*Q/Q0 * |
|---|---|---|
| 0.19 | 0.103 | 3.46 |
| 0.98 | 0.154 | 5.16 |
| 2.01 | 0.172 | 5.78 |
| 6.94 | 0.194 | 6.53 |
| 11.03 | 0.212 | 7.14 |
| 15.93 | 0.244 | 8.20 |
| 17.92 | 0.253 | 8.49 |
| 24.98 | 0.264 | 8.88 |
| 35.20 | 0.279 | 9.39 |
| 45.92 | 0.342 | 11.49 |
| 52.23 | 0.304 | 10.22 |
| 60.20 | 0.316 | 10.63 |
| 73.21 | 0.417 | 14.01 |
| 77.14 | 0.417 | 14.01 |
| 91.13 | 0.439 | 14.75 |
| 115.93 | 0.585 | 19.66 |
| 150.18 | 0.572 | 19.22 |

* 0,6 partie d'un empâtage d'acide hexachloroplatinique titrant 0,18% en poids de platine métal.

La composition catalysée se détache aisément des cylindres du mélangeur. Elle est ensuite extrudée en un boudin continu de 26 mm de diamètre qui est ensuite durci par passage dans un four de 6 m porté à 350°C à la vitesse de 1 m par minute. On obtient un jonc uniforme qui est ensuite recuit à 150°C pendant 4 heures.

On découpe ensuite un cylindre dont les caractéristiques sont les suivantes :

* Diamètre : 25,9 mm
* Hauteur : 33,6 mm
* Surface : 37,9 cm$^2$
* Volume : 17,70 cm$^3$
* S/V : 2,14 cm$^{-1}$
* Masse totale : 19,35 g
* Quantité initiale de I(Qo) : 3,27 g

Le dit cylindre est immergé dans 500 ml d'eau distillée, thermostatée à 20°C.

Le tableau 3 rassemble les résultats de la cinétique d'élution. La courbe Q/Qo = f(t), ainsi que le Te, sont reportés sur la figure 1.

Exemple 4.

Composition : on mélange intimement à l'aide d'un malaxeur les ingrédients suivants :
* 100 parties d'une gomme (A) diméthylméthylvinylpolysiloxane bloquée à chacune de ses deux extrémités par un motif triméthylsiloxy et comprenant dans sa chaine 99,8% molaire de motif diméthylsiloxy et 0,2% molaire de motifs vinylméthylsiloxy et de viscosité 10 millions de mPa · s à 25°C.
* 43,5 parties de charge (B) qui est un silice de combustion traité D4 (octaméthyltétracyclosiloxane) de surface spécifique BET de 300 m2/g.

TABLEAU 3

| TEMPS (JOUR) | Q cumulé (gramme) ion acti | 100*Q/Q0 % |
|---|---|---|
| 0.19 | 0.177 | 5.42 |
| 0.98 | 0.291 | 8.89 |
| 2.01 | 0.341 | 10.42 |
| 6.94 | 0.369 | 11.27 |
| 11.03 | 0.388 | 11.84 |
| 15.95 | 0.411 | 12.55 |
| 17.92 | 0.458 | 14.00 |
| 24.98 | 0.449 | 13.70 |
| 35.20 | 0.468 | 14.28 |
| 45.92 | 0.558 | 17.03 |
| 52.23 | 0.468 | 14.29 |
| 60.20 | 0.544 | 16.61 |
| 73.21 | 0.701 | 21.39 |
| 77.15 | 0.701 | 21.42 |
| 91.13 | 0.705 | 21.51 |
| 115.93 | 0.901 | 27.50 |
| 150.18 | 0.886 | 27.07 |

* 1 partie d'un diméthylpolysiloxane linéaire bloqué à ses deux extrémités par des groupements diméthylhydroxysiloxane de viscosité 50 mPa · s.

* 0,2 partie d'octométhyltétracyclosiloxane).

* 37,2 parties de KIO3 de granulométrie moyenne égale à 5 μm (C)

Le malaxage est arrêté 30 minutes après la fin de l'introduction de la silice. On enlève du malaxeur la composition homogène venant d'être préparée et qui est appelée le mélange maître (MM).

On transfère le MM sur un mélangeur à cylindres pour incorporer pour 100 parties de MM :

* 0,43 partie d'un méthylhydrogénosiloxane linéaire de viscosité 45 mPa · s à 25°C, bloqué à chaque extrémité de sa chaine par un motif triméthylsiloxy, comportant dans sa chaine essentiellement des motifs hydrogénosiloxy.

* 0,6 partie d'un empâtage d'acide hexachloroplatinique titrant 0,18% en poids de platine métal.

La composition catalysée se détache aisément des cylindres du mélangeur. Elle est ensuite extrudée en un boudin continu de 23 mm de diamètre qui est ensuite durci par passage dans un four de 6 m porté à 350°C à la vitesse de 1 m par minute. On obtient un jonc uniforme qui est ensuite recuit à 150°C pendant 4 heures.

On découpe ensuite un cylindre dont les caractéristiques sont les suivantes :

| | |
|---|---|
| Diamètre : | 22,6 mm |
| Hauteur : | 52,1 mm |
| Surface : | 45,0 cm$^2$ |
| Volume : | 20,90 cm$^3$ |
| S/V : | 2,14 cm$^{-1}$ |
| Masse totale : | 25,65 g |
| Quantité initiale de I(Qo) : | 3,04 g |

Le dit cylindre est immergé dans 500 ml d'eau distillée, thermostatée à 20°C.

Le tableau 4 rassemble les résultats de la cinétique d'élution. La courbe Q/Qo = f(t), ainsi que le Te, sont reportés sur la figure 2.

Exemple 5.

On utilise la même composition qu'à l'exemple 1 sauf que l'on incorpore 37,2 parties de KIO$_3$ de granulométrie comprise entre 100 et 200 μm.

Le malaxage est arrêté 30 minutes après la fin de l'introduction de la silice. On enlève du malaxeur la composition homogène venant d'être préparée et qui est appelée le mélange maître (MM).

On transfère le MM sur un mélangeur à cylindres pour incorporer pour 100 parties de MM :

* 0,43 partie d'un méthylhydrogénosiloxane linéaire de viscosité 45 mPa · s à 25°C, bloqué à chaque extrémité de sa chaine par un motif triméthylsiloxy, comportant dans sa chaine essentiellement des motifs hydrogénosiloxy.

* 0,6 partie d'un empâtage d'acide hexachloroplatinique titrant 0,18% en poids de platine métal.

La composition catalysée se détache aisément des cylindres du mélangeur. Elle est ensuite extrudée en un boudin continu de 21 mm de diamètre qui est ensuite durci par passage dans un four de 6 m porté à 350°C à la vitesse de 1 m par minute. On obtient un jonc uniforme qui est ensuite recuit à 150°C pendant 4 heures.

On découpe ensuite un cylindre dont les caractéristiques sont les suivantes :

| | |
|---|---|
| * Diamètre : | 21,4 mm |
| * Hauteur : | 81,0 mm |
| * Surface : | 61,6 cm$^2$ |
| * Volume : | 29,13 cm$^3$ |
| * S/V : | 2,12 cm$^{-1}$ |
| * Masse totale : | 37,45 g |
| * Quantité initiale de I(Qo) : | 4,45 g |

TABLEAU 4

| TEMPS (JOUR) | Q cumulé (gramme) ion acti | 100*Q/Q0 % |
|---|---|---|
| 0.19 | 0.014 | 0.45 |
| 0.98 | 0.025 | 0.82 |
| 2.01 | 0.034 | 1.11 |
| 6.91 | 0.055 | 1.80 |
| 11.01 | 0.059 | 1.93 |
| 15.92 | 0.076 | 2.50 |
| 17.91 | 0.080 | 2.63 |
| 24.97 | 0.084 | 2.77 |
| 35.19 | 0.096 | 3.14 |
| 45.91 | 0.128 | 4.22 |
| 52.21 | 0.119 | 3.90 |
| 60.16 | 0.132 | 4.33 |
| 73.20 | 0.173 | 5.69 |
| 77.12 | 0.180 | 5.90 |
| 91.12 | 0.184 | 6.05 |
| 115.92 | 0.237 | 7.79 |
| 150.18 | 0.243 | 7.98 |

Le dit cylindre est immergé dans 500 ml d'eau distillée, thermostatée à 20°C.

Le tableau 5 rassemble les résultats de la cinétique d'élution. La courbe Q/Qo = f(t), ainsi que le Te, sont reportés sur la figure 2.

Exemple 6.

On utilise la même composition qu'à l'exemple 1 sauf que l'on incorpore 37,2 parties de KIO$_3$ de granulo-métrie comprise entre 200 et 400 µm.

Le malaxage est arrêté 30 minutes après la fin de l'introduction de la silice. On enlève du malaxeur la composition homogène venant d'être préparée et qui est appelée le mélange maître (MM).

On transfère le MM sur un mélangeur à cylindres pour incorporer pour 100 parties de MM :

\* 0,43 partie d'un méthylhydrogénosiloxane linéaire de viscosité 45 mPa · s à 25°C, bloqué à chaque extrémité de sa chaine par un motif triméthylsiloxy, comportant dans sa chaine essentiellement des motifs hydrogénosiloxy.

\* 0,6 partie d'un empâtage d'acide hexachloroplatinique titrant 0,18% en poids de platine métal.

La composition catalysée se détache aisément des cylindres du mélangeur. Elle est ensuite extrudée en un boudin continu de 23 mm de diamètre qui est ensuite durci par passage dans un four de 6 m porté à 350°C à la vitesse de 1 m par minute. On obtient un jonc uniforme qui est ensuite recuit à 150°C pendant 4 heures.

On découpe ensuite un cylindre dont les caractéristiques sont les suivantes :

| | |
|---|---|
| \* Diamètre : | 23,6 mm |
| \* Hauteur : | 46,7 mm |
| \* Surface : | 43,4 cm$^2$ |
| \* Volume : | 20,42 cm$^3$ |
| \* S/V : | 2,2 cm$^{-1}$ |
| \* Masse totale : | 26,75 g |
| \* Quantité initiale de I(Qo) : | 3,175 g |

TABLEAU 5

| TEMPS (JOUR) | Q cumulé (gramme) ion acti | 100*Q/Q0 % |
|---|---|---|
| 0.19 | 0.070 | 1.58 |
| 0.98 | 0.103 | 2.32 |
| 2.01 | 0.115 | 2.59 |
| 6.91 | 0.120 | 2.70 |
| 11.01 | 0.126 | 2.84 |
| 15.92 | 0.139 | 3.13 |
| 17.91 | 0.145 | 3.26 |
| 24.97 | 0.143 | 3.23 |
| 35.19 | 0.157 | 3.54 |
| 45.91 | 0.185 | 4.17 |
| 52.21 | 0.190 | 4.28 |
| 60.17 | 0.184 | 4.14 |
| 73.20 | 0.229 | 5.15 |
| 77.12 | 0.238 | 5.36 |
| 91.13 | 0.238 | 5.36 |
| 115.92 | 0.289 | 6.50 |
| 150.18 | 0.238 | 5.36 |

Le dit cylindre est immergé dans 500 ml d'eau distillée, thermostatée à 20°C.

Le tableau 6 rassemble les résultats de la cinétique d'élution. La courbe Q/Qo = f(t), ainsi que le Te, sont reportés sur la figure 2.

TABLEAU 6

| TEMPS (JOUR) | Q cumulé (gramme) ion acti | 100*Q/Q0 % |
|---|---|---|
| 0.19 | 0.078 | 2.44 |
| 0.98 | 0.139 | 4.38 |
| 2.01 | 0.179 | 5.64 |
| 6.91 | 0.194 | 6.12 |
| 11.01 | 0.204 | 6.43 |
| 15.92 | 0.208 | 6.55 |
| 17.92 | 0.216 | 6.79 |
| 24.97 | 0.207 | 6.52 |
| 35.20 | 0.203 | 6.41 |
| 45.91 | 0.227 | 7.16 |
| 52.22 | 0.231 | 7.29 |
| 60.17 | 0.209 | 6.57 |
| 73.21 | 0.258 | 8.14 |
| 77.12 | 0.268 | 8.46 |
| 91.13 | 0.253 | 7.96 |
| 115.92 | 0.300 | 9.46 |
| 150.18 | 0.263 | 8.30 |

## Revendications

1. Composition de silicone vulcanisable à chaud comportant (A) une gomme diorganopolysiloxane, présentant par molécule au moins deux groupes vinyle liés au silicium et une viscosité à 25°C d'au moins 500000 mPa · s, (B) au moins un organohydrogénopolysiloxane présentant par molécule au moins trois atomes d'hydrogène liés au silicium, (C) une charge renforçante, (D) une quantité catalytiquement efficace d'un catalyseur qui est un composé d'un métal du groupe du platine, (E) au moins un composé de l'iode à l'exclusion de l'iode moléculaire $I_2$ solide à la température ambiante, soluble dans l'eau, non toxique, dispersé de façon homogène dans la composition.

2. Composition de silicone selon la revendication 1, caractérisée en ce que le composé (C) est une charge renforçante siliceuse.

3. Composition de silicone selon la revendication 1, caractérisée en ce que le composé de l'iode (E) est un iodure ou iodate de formules générales :

$$(M^{a+})\,(I^-)a$$

et $$(M^{a+})\,(IO^-_3)_a$$

dans lesquelles a est un nombre entier supérieur ou égal à 1 et M est un cation choisi parmi un métal alcalin, un métal alcalino-terreux, un métal de transition et un ammonium quaternaire $(NY_4)^+$ dont les radicaux Y identiques ou différents représentent un radical alkyle linéaire ou ramifié en $C_1$-$C_{20}$ ou un atome d'hydrogène.

4. Composition selon la revendication 1, caractérisée en ce que le composé de l'iode (C) est choisi parmi:

| | |
|---|---|
| $NaI$, | $NaIO_3$, |
| $KI$, | $KIO_3$, |
| $MgI_2$, | $MgI_2 \cdot 8H_2O$, |

$Mg(IO_3)_2 \cdot 4H_2O$,
$NH_4I$
$FeI_2 \cdot 4H_2O$
$MnI_2$

5. Composition selon l'une quelconque des revendications précédentes, caractérisée en ce qu'elle comporte :

— (A) : 100 parties en poids d'une gomme diorganopolysiloxane selon la revendication 1,

— (B) : au moins un organohydrogénopolysiloxane présentant par molécule au moins 3 atomes d'hydrogène liés au silicium en quantité telle que le rapport en nombre des fonctions hydrures de (B) sur les groupes vinyle de (A) soit compris entre 0,4 et 10,

— (C) : 5 à 130 parties en poids d'une charge renforçante de préférence siliceuse choisie parmi les silices de pyrogénation et les silices de précipitation,

— (D) : une quantité catalytiquement efficace d'un catalyseur qui est un composé d'un métal du groupe du platine, et

— (E) : 5 à 150 parties en poids du composé de l'iode.

6. Composition selon l'une quelconque des revendications précédentes, caractérisée en ce que la gomme (A) a pour formule générale $R_{3-a}(R'O)_aSiO(R_2SiO)nSi(OR')_aR_{3-a}$ dans laquelle les symboles R, identiques ou différents, représentent des radicaux hydrocarbonés en $C_1$-$C_8$, substitués ou non par des atomes d'halogène, des radicaux cyano ; le symbole R' représente un atome d'hydrogène, un radical alkyle en $C_1$-$C_4$, le symbole a représente zéro ou un, le symbole n représente un nombre ayant une valeur suffisante pour obtenir une viscosité d'au moins 2 Millions de mPa · s à 25°C, au moins 50% en nombre de radicaux représentés par R sont des radicaux méthyle, et en ce que 0,005 à 0,5 mole % des motifs entrant dans la constitution de la gomme (A) sont choisis parmi ceux de formules $(CH_2 = CH)(R)SiO$ et $(CH_2 = CH)R_{2-a}(RO')_aSi_{0,5}$

7. Composition selon l'une quelconque des revendications 1 à 6, caractérisée en ce qu'elle comporte de 0,1 à 6 parties d'un organohydrogénopolysiloxane (B) présentant un motif siloxane de formule générale moyenne :

$$(H)_c\ (R'')_d\ RSiO\underline{\frac{4-d-c}{2}}$$

dans laquelle R'' représente des radicaux méthyle, phényle et vinyle, 50% au moins de ces radicaux étant des radicaux méthyle, c représente un nombre quelconque de 0,01 à 1 inclus et d représente un nombre quelconque de 0,01 à 2 inclus.

8. Composition selon l'une quelconque des revendications précédentes, caractérisée en ce que les organohydrogénopolysiloxanes sont choisis parmi les polymères linéaires, ramifiés ou cycliques constitués de motifs choisis parmi ceux de formules :

$R''_2SiO,\ H(R'')SiO,\ H(R'')_2SiO_{0,5},\ HSiO_{0,5},\ R''SiO_{1,5},\ SiO_2,\ R_3SiO_{0,5}$

et sont ajoutés en quantité telle que le rapport en nombre des fonctions hydrure de (B) sur les groupes vinyle (A) soit compris entre 1,1 et 4.

9. Composition selon l'une quelconque des revendications précédentes, caractérisée en ce que jusqu'à 90% en poids de la charge silicone renforçante (C) est remplacée par des charges semi-renforçantes ou de bourrage dont le diamètre particulaire est supérieur à 0,1 micron.

10. Composition selon l'une quelconque des revendications précédentes, durcie en un élastomère par chauffage.

11. Procédé de traitement d'eaux, caractérisé en ce qu'on immerge une quantité adaptée d'une composition durcie telle que définie à la revendication 10 en vue de libérer dans l'eau une quantité continue et contrôlée de composé iodé.

## Patentansprüche

1. In der Wärme vulkanisierbare Siliconzusammensetzung, enthaltend (A) ein Diorganopolysiloxangummi, das je Molekül zumindest zwei an das Silicium gebundene Vinylgruppen aufweist, mit einer Viskosität bei 25°C von zumindest 500000 mPa · s, (B) zumindest ein Organohydrogenopolysiloxan, das je Molekül zumindest drei an das Silicium gebundene Wasserstoffatome aufweist, (C) einen verstärkenden Füllstoff, (D) eine katalytisch wirksame Menge eines Katalysators, der eine Verbindung eines Metalls der Platingruppe ist, (E) zumindest eine Jodverbindung unter Ausschluß von molekularem Jod $J_2$, die bei Raumtemperatur fest, in Wasser löslich, nicht-toxisch und in der Zusammensetzung homogen dispergiert ist.

2. Siliconzusammensetzung gemäß Anspruch 1, dadurch gekennzeichnet, daß die Verbindung (C) ein silicium-haltiger, verstärkender Füllstoff ist.

3. Siliconzusammensetzung gemäß Anspruch 1, dadurch gekennzeichnet, daß die Jodverbindung (E) ein Jodid oder Jodat der allgemeinen Formeln

$$(M^{a+}) (J^-)_a$$
und $\quad (M^{a+}) (JO_3^-)_a$

ist, worin a eine ganze Zahl höher als oder gleich 1 ist und M für ein Kation steht, ausgewählt unter einem Alkalimetall, einem Erdalkalimetall, einem Übergangsmetall und einem quaternären Ammonium $(NY_4)^+$, dessen Reste Y, die gleich oder verschieden sind, einen linearen oder verzweigten $C_{1-20}$-Alkylrest oder ein Wasserstoffatom bedeuten.

4. Zusammensetzung gemäß Anspruch 1, dadurch gekennzeichnet, daß die Jodverbindung (C) ausgewählt ist unter

| | |
|---|---|
| NaJ, | $NaJO_3$, |
| KJ, | $KJO_3$, |
| $MgJ_2$, | $MgJ_2 \cdot 8H_2O$, |
| $Mg(JO_3)_2 \cdot 4H_2O$, | |
| $NH_4J$ | |
| $FeJ_2 \cdot 4H_2O$ | |
| $MnJ_2$. | |

5. Zusammensetzung gemäß einem der vorhergehenden Ansprüche, dadurch gekennzeichnet, daß sie enthält

(A) 100 Gew.Teile eines Diorganopolysiloxangummis gemäß Anspruch 1,

(B) zumindest ein Organohydrogenopolysiloxan, das je Molekül zumindest drei an das Silicium gebundene Wasserstoffatome in einer derartigen Menge besitzt, daß das Zahlenverhältnis der Hydridfunktionen von (B) zu den Vinylgruppen von (A) zwischen 0,4 und 10 beträgt,

(C) 5 bis 130 Gew.Teile eines verstärkenden, vorzugsweise siliciumhaltigen Füllstoffs, ausgewählt unter den Pyrolyse-Siliciumdioxiden und den Ausfällungs-Siliciumdioxiden,

(D) eine katalytisch wirksame Menge eines Katalysators, der eine Verbindung eines metalls der Platingruppe ist, und

(E) 5 bis 150 Gew.Teile Jodverbindung.

6. Zusammensetzung gemäß einem der vorhergehenden Ansprüche, dadurch gekennzeichnet, daß das Gummi (A) die allgemeine Formel $R_{3-a}(R'O)_aSiO(R_2SiO)_nSi(OR')_aR_{3-a}$ besitzt, worin die Symbole R, die gleich oder verschieden sind, $C_{1-8}$-Kohlenwasserstoffreste, substituiert oder nicht durch Halogenatome, Cyanogruppen, bedeuten ; das Symbol R' für ein Wasserstoffatom, einen $C_{1-4}$-Alkylrest steht ; das Symbol a für 0 oder 1 steht ; das Symbol n eine ganze Zahl mit einem ausreichenden Wert wiedergibt, um zu einer Viskosität von zumindest 2 Millionen mPa · s bei 25°C zu gelangen, wobei zumindest 50% der Anzahl der durch R wiedergegebenen Reste Methylreste sind, und daß 0,005 bis 0,5 Mol-% der in die Konstitution des Gummis (A) eingehenden Gruppen unter denjenigen der Formeln $(CH_2 = CH)(R)SiO$ und $(CH_2 = CH)R_{2-a}(RO')_aSi_{0,5}$ ausgewählt sind.

7. Zusammensetzung gemäß einem der Ansprüche 1 bis 6, dadurch gekennzeichnet, daß sie 0,1 bis 6 Teile eines Organohydrogenopolysiloxans (B) mit einer Siloxangruppe der durchschnittlichen allgemeinen Formel

$$(H)_c \ (R'')_d \ RSiO_{\frac{4-d-c}{2}}$$

enthält, worin R" Methyl-, Phenyl- und Vinylreste bedeutet, wobei zumindest 50% dieser Reste Methylreste sind, c für eine beliebige Zahl von 0,01 bis 1 einschließlich steht und d eine beliebige Zahl von 0,01 bis 2 einschließlich bedeutet.

8. Zusammensetzung gemäß einem der vorhergehenden Ansprüche, dadurch gekennzeichnet, daß die Organohydrogenopolysiloxane ausgewählt sind unter den linearen, verzweigten oder cyclischen Polymeren, bestehend aus Gruppen, ausgewählt unter denjenigen der Formeln

$R''_2SiO$, $H(R'')SiO$, $H(r'')_2SiO_{0,5}$, $HSiO_{0,5}$, $R''SiO_{1,5}$, $SiO_2$, $R_3SiO_{0,5}$

und in einer derartigen Menge zugegeben werden, daß das Zahlenverhältnis der Hydridfunktionen von (B) zu den Vinylgruppen von (A) zwischen 1,1 und 4 liegt.

9. Zusammensetzung gemäß einem der vorhergehenden Ansprüche, dadurch gekennzeichnet, daß bis zu 90 Gew.% des verstärkenden Siliconfüllstoffs (C) durch halbverstärkende Füllstoffe oder Füllmaterial ersetzt sind, deren Teilchendurchmesser höher als 0,1 Mikron ist.

10. Zusammensetzung gemäß einem der vorhergehenden Ansprüche durch Erhitzen zu einem Elastomeren gehärtet.

11. Verfahren zur Behandlung von Wässern, dadurch gekennzeichnet, daß man eine geeignete Menge einer gehärteten Zusammensetzung, wie in Anspruch 10 definiert, eintaucht, um in das Wasser eine kontinuierliche und kontrollierte Menge der Jodverbindung freizusetzen.

## Claims

1. Hot-vulcanisable silicone composition comprising (A) a diorganopolysiloxane resin containing, per molecule, at least two vinyl groups bonded to silicon and a viscosity at 25°C of at least 500,000 mPa s, (B) at least one organohydropolysiloxane containing, per molecule, at least three hydrogen atoms bonded to silicon, (C) a reinforcing filler, (D) a catalytically effective quantity of a catalyst which is a compound of a metal of the platinum group, and (E) at least one iodine compound, with the exception of molecular iodine $I_2$, which is solid at room temperature, soluble in water, nontoxic and homogeneously dispersed in the composition.

2. Silicone composition according to Claim 1, characterised in that the compound (C) is a siliceous reinforcing filler.

3. Silicone composition according to Claim 1, characterised in that the iodine compound (E) is an iodide or iodate of general formulae :

$(M^{a+})$     $(I^-)_a$
and $(M^{a+})$     $(IO^-_3)_a$

in which formulae a is an integer greater than or equal to 1 and M is a cation chosen from an alkali metal, an alkaline earth metal, a transition metal and a quaternary ammonium $(NY_4)^+$, in which the radicals Y, which may be identical or different, represent a $C_1$-$C_{20}$ straight-chain or branched alkyl radical or a hydrogen atom.

4. Composition according to Claim 1, characterised in that the iodine compound (E) is chosen from :

| | |
|---|---|
| NaJ, | $NaJO_3$, |
| KJ, | $KJO_3$, |
| $MgJ_2$, | $MgJ_2 \cdot 8H_2O$, |

Mg $(JO_3)_2 \cdot 4H_2O$,
$NH_4J$
$FeJ_2 \cdot 4H_2O$.
$MnJ_2$.

5. Composition according to any one of the preceding claims, characterised in that it comprises :
— (A) : 100 parts by weight of a diorganopolysiloxane resin according to Claim 1,
— (B) : at least one organohydropolysiloxane containing, per molecule, at least 3 hydrogen atoms bonded to silicon in a quantity such that the ratio of the number of hydride functional groups in (B) to the vinyl groups in (A) is between 0.4 and 10,
— (C) : 5 to 130 parts by weight of a reinforcing filler which is preferably siliceous chosen from pyrogenic silicas and precipitated silicas,
— (D) : a catalytically effective quantity of a catalyst which is a compound of a metal of the platinum group, and
— (E) : 5 to 150 parts by weight of the iodine compound.

6. Composition according to any one of the preceding claims, characterised in that the resin (A) has the general formula $R_{3-a}(R'O)_aSiO(R_2SiO)_nSi(OR')_aR_{3-a}$, in which the symbols R, which may be identical or different, represent $C_1$-$C_8$ hydrocarbon radicals which are unsubstituted or substituted by halogen atoms or cyano radicals ; the symbol R' represents a hydrogen atom or a $C_1$-$C_4$ alkyl radical, the symbol a represents zero or one, the symbol n represents a number having a value sufficient to obtain a viscosity of at least 2 million mPa · s at 25°C, and at least 50% of the number of the radicals represented by R are methyl radicals, and in that 0.005 to 0.5 mol% of the units present in the structure of the resin (A) are chosen from those of formulae $(CH_2 = CH)(R)SiO$ and $(CH2 = CH)R_{2-a}(RO')_aSi_{0.5}$.

7. Composition according to any one of Claims 1 to 6, characterised in that it comprises from 0.1 to 6 parts of an organohydropolysiloxane (B) having a siloxane unit of average general formula :

$$(H)_c \ (R'')_d \ RSiO_{\frac{4-d-c}{2}}$$

in which R'' represents methyl, phenyl and vinyl radicals, at least 50% of these radicals being methyl radicals, c represents any number from 0.01 to 1 inclusive and d represents any number from 0.01 to 2 inclusive.

8. Composition according to any one of the preceding claims, characterised in that the organohydropolysiloxanes are chosen from straight-chain, branched or cyclic polymers consisting of units chosen from those of formulae :

$R''_2SiO, H(R'')SiO, H(R'')_2SiO_{0.5}, HSiO_{0.5}, R''SiO_{1.5}, SiO_2, R_3SiO_{0.5}$

and are added in a quantity such that the ratio of the number of the hydride functional groups in (B) to the vinyl groups in (A) is between 1.1 and 4.

9. Composition according to any one of the preceding claims, characterised in that up to 90% by weight of the reinforcing silicone filler (C) has been replaced by semireinforcing or packing fillers whose particle diameter is more than 0.1 micron.

10. Composition according to any one of the preceding claims, cured to form an elastomer by heating.

11. Water treatment process, characterised in that a suitable quantity of a cured composition as defined in Claim 10 is immersed with a view to releasing a continuous and controlled quantity of iodine compound into the water.

FIGURE 1

## FIGURE 2

EP 0 283 407 B1